# EUROPÄISCHE PATENTSCHRIFT

(11) **EP 3 283 562 B1**
(45) Veröffentlichungstag und Bekanntmachung des Hinweises auf die Patenterteilung: **13.02.2019**
(21) Anmeldenummer: 16719276.4
(22) Anmeldetag: 13.04.2016
(51) Int. Cl.: C08K 5/29, C08L 77/00

(54) **POLYAMIDE MIT BESSEREN OPTISCHEN EIGENSCHAFTEN**
POLYAMIDES WITH IMPROVED OPTICAL PROPERTIES
POLYAMIDE PRÉSENTANT DES CARACTÉRISTIQUES OPTIQUES AMÉLIORÉES

(30) Priorität: 16.04.2015 EP 15163880
(43) Veröffentlichungstag der Anmeldung: 21.02.2018
(73) Patentinhaber: BASF SE, 67056 Ludwigshafen (DE)
(72) Erfinder: RICHTER, Florian, 68199 Mannheim (DE); XALTER, Rainer, 69115 Heidelberg (DE); PARK, Hye Jin, 49076 Osnabrück (DE); DABBOUS, Raphael, 4125 Riehen (CH)
(74) Vertreter: BASF IP Association
(86) Internationale Anmeldenummer: PCT/EP2016/058086
(87) Internationale Veröffentlichungsnummer: WO 2016/166140

(56) Entgegenhaltungen:
- WO-A1-2013/139802
- US-A- 4 600 752
- US-A- 4 918 148
- US-A1- 2005 143 548

## Beschreibung

Die Erfindung betrifft die Verwendung von thermoplastischen Formmassen, enthaltend
A) 30 bis 99 Gew.-% eines thermoplastischen Polyamids
B) 0,01 bis 10 Gew.-% eines organischen Isocyanates oder
   Diisocyanates oder deren Mischungen
C) 0 bis 60 Gew.-% weiterer Zusatzstoffe,
wobei die Summe der Gewichtsprozente A) bis C) 100 % ergibt,
zur Herstellung von Formkörpern jeglicher Art Art mit verbessertem Haze (gemessen nach ASTM D1003) und/oder verbesserter Clarity (gemessen nach ASTM D1003) und/oder erhöhter Lasertransparenz (gemessen bei einer Wellenlänge von 1064 nm mittels einer thermoelektrischen Leistungsmessung).

Weiterhin betrifft die Erfindung die Verwendung von transparenten Formkörpern und/oder mit verminderter Trübung zur Herstellung von Formkörpern jeglicher Art, insbesondere mittels Laserdurchstrahlschweißen und die Verwendung derartiger Formkörper in unterschiedlichen Anwendungsgebieten.

Polyamide werden in sehr verschiedenen Anwendungen eingesetzt, z.B. für Kfz, elektrische, elektronische Bauteile und als Verpackungsmaterial für Lebensmittel.

Platten, Filme, Container, Scheinwerfer und ähnliche Bauteile erfordern größere Transparenz (insbesondere Lasertransparenz) und verringerte Trübung für bestimmte Anwendungsgebiete.

Aus der WO 2013/139802 ist bekannt, Harnstoffderivate als Additive zur Verbesserung der optischen Eigenschaften von Polyamiden einzusetzen.

Diisocyanate bzw. Isocyanate in Polyamiden einzusetzen, ist u.a. aus JP48000995 und US3668171 bekannt. Die Verbesserung optischer Eigenschaften wird nicht erwähnt.

Die US2005/143548 beschreibt ein Verfahren zur Herstellung eines hochmolekularen Polyamids bei dem ein niedermolekulares Polyamid in der Schmelze mit einem geblockten Diisocyanat vermischt wird. Im Gegensatz zum Einsatz von nicht-geblockten Diisocyanaten kommt es beim Einsatz von geblockten Diisocyanaten zu einer geringeren Verfärbung des Polymers. Ein positiver Einfluss auf die in der vorliegenden Erfindung beanspruchten optischen Eigenschaften Haze, Clarity und Lasertransparenz wird in der US2005143548 nicht erwähnt. Aufgabe der vorliegenden Erfindung war es daher, die optischen Eigenschaften Klarheit (Trübung) und/oder Transparenz (insbesondere Lasertransparenz) bei Polyamiden zu verbessern. Überraschenderweise wird diese Aufgabe durch Zugabe der erfindungsgemäßen Isocyanate und/oder Diisocyanate zu Polyamiden gelöst.

Demgemäß wurde die eingangs definierte Verwendung der Formmassen gefunden. Bevorzugte Ausführungsformen können den Unteransprüchen entnommen werden.

Als Komponente A) enthalten die erfindungsgemäßen Formmassen 30 bis 99, vorzugsweise 30 bis 98 und insbesondere 30 bis 90 Gew.-% mindestens eines Polyamides.

Die Polyamide der erfindungsgemäßen Formmassen weisen im allgemeinen eine Viskositätszahl von 90 bis 350, vorzugsweise 110 bis 240 ml/g auf, bestimmt in einer 0,5 gew.-%igen Lösung in 96 gew.-%iger Schwefelsäure bei 25°C gemäß ISO 307.

Halbkristalline oder amorphe Harze mit einem Molekulargewicht (Gewichtsmittelwert) von mindestens 5.000, wie sie z.B. in den amerikanischen Patentschriften 2 071 250, 2 071 251, 2 130 523, 2 130 948, 2 241 322, 2 312 966, 2 512 606 und 3 393 210 beschrieben werden, sind bevorzugt.

Beispiele hierfür sind Polyamide, die sich von Lactamen mit 7 bis 13 Ringgliedern ableiten, wie Polycaprolactam, Polycapryllactam und Polylaurinlactam sowie Polyamide, die durch Umsetzung von Dicarbonsäuren mit Diaminen erhalten werden.

Als Dicarbonsäuren sind Alkandicarbonsäuren mit 6 bis 12, insbesondere 6 bis 10 Kohlenstoffatomen und aromatische Dicarbonsäuren einsetzbar. Hier seien nur Adipinsäure, Azelainsäure, Sebacinsäure, Dodecandisäure und Terephthal- und/oder Isophthalsäure als Säuren genannt.

Als Diamine eignen sich besonders Alkandiamine mit 6 bis 12, insbesondere 6 bis 8 Kohlenstoffatomen sowie m-Xylylendiamin (z.B. Ultramid® X17 der BASF SE, ein 1:1 molares Verhältnis von MXDA mit Adipinsäure), Di-(4-aminophenyl)methan, Di-(4-amino-cyclohexyl)-methan, 2,2-Di- (4-aminophenyl)-propan, 2,2-Di-(4-aminocyclohexyl)-propan oder 1,5-Diamino-2-methylpentan.

Bevorzugte Polyamide sind Polyhexamethylenadipinsäureamid, Polyhexamethylen-sebacinsäureamid und Polycaprolactam sowie Copolyamide 6/66, insbesondere mit einem Anteil von 5 bis 95 Gew.-% an Caprolactam-Einheiten (z.B. Ultramid®C31 der BASF SE).

Weiterhin geeignete Polyamide sind erhältlich aus ω-Aminoalkylitrilen wie beispielsweise Aminocapronitril (PA 6) und Adipodinitril mit Hexamethylendiamin (PA 66) durch sog. Direktpolymerisation in Anwesenheit von Wasser, wie beispielsweise in der DE-A 10313681, EP-A 1198491 und EP 922065 beschrieben.

Außerdem seien auch noch Polyamide erwähnt, die z.B. durch Kondensation von 1,4-Diaminobutan mit Adipinsäure unter erhöhter Temperatur erhältlich sind (Polyamid 4,6). Herstellungsverfahren für Polyamide dieser Struktur sind z.B. in den EP-A 38 094, EP-A 38 582 und EP-A 39 524 beschrieben.

Weiterhin sind Polyamide, die durch Copolymerisation zweier oder mehrerer der vorgenannten Monomeren erhältlich sind, oder Mischungen mehrerer Polyamide geeignet, wobei das Mischungsverhältnis beliebig ist. Besonders bevorzugt sind Mischungen von Polyamid 66 mit anderen Polyamiden, insbesondere Copolyamide 6/66.

Weiterhin haben sich solche teilaromatischen Copolyamide wie PA 6/6T und PA 66/6T als besonders vorteilhaft erwiesen, deren Triamingehalt weniger als 0,5, vorzugsweise weniger als 0,3 Gew.-% beträgt (siehe EP-A 299 444). Weitere hochtemperaturbeständige Polyamide sind aus der EP-A 19 94 075 bekannt (PA 6T/6I/MXD6).

Die Herstellung der bevorzugten teilaromatischen Copolyamide mit niedrigem Triamingehalt kann nach den in den EP-A 129 195 und 129 196 beschriebenen Verfahren erfolgen.

Die nachfolgende nicht abschließende Aufstellung enthält die genannten, sowie weitere Polyamide A) im Sinne der Erfindung und die enthaltenen Monomeren.
AB-Polymere:
   - PA 4: Pyrrolidon
   - PA 6: ε-Caprolactam
   - PA 7: Ethanolactam
   - PA 8: Capryllactam
   - PA 9: 9-Aminopelargonsäure
   - PA 11: 11-Aminoundecansäure
   - PA 12: Laurinlactam
AA/BB-Polymere
   - PA 46: Tetramethylendiamin, Adipinsäure
   - PA 66: Hexamethylendiamin, Adipinsäure
   - PA 69: Hexamethylendiamin, Azelainsäure
   - PA 610: Hexamethylendiamin, Sebacinsäure
   - PA 612: Hexamethylendiamin, Decandicarbonsäure
   - PA 613: Hexamethylendiamin, Undecandicarbonsäure
   - PA 1212: 1,12-Dodecandiamin, Decandicarbonsäure
   - PA 1313: 1,13-Diaminotridecan, Undecandicarbonsäure
   - PA 6T: Hexamethylendiamin, Terephthalsäure
   - PA 9T: 1,9-nonanediamin, Terephthalsäure
   - PA MXD6: m-Xylylendiamin, Adipinsäure
   - PA 6I: Hexamethylendiamin, Isophthalsäure
   - PA 6-3-T: Trimethylhexamethylendiamin, Terephthalsäure
   - PA 6/6T: (siehe PA 6 und PA 6T)
   - PA 6/66: (siehe PA 6 und PA 66)
   - PA 6/12: (siehe PA 6 und PA 12)
   - PA 66/6/610: (siehe PA 66, PA 6 und PA 610)
   - PA 6I/6T: (siehe PA 6I und PA 6T)
   - PA PACM 12: Diaminodicyclohexylmethan, Laurinlactam
   - PA 6I/6T/PACM: wie PA 6I/6T + Diaminodicyclohexylmethan
   - PA 12/MACMI: Laurinlactam, Dimethyl-diaminodicyclohexylmethan, Isophthalsäure
   - PA 12/MACMT: Laurinlactam, Dimethyl-diaminodicyclohexylmethan, Terephthalsäure
   - PA PDA-T: Phenylendiamin, Terephthalsäure

Als Komponente B) enthalten die erfindungsgemäß verwendbaren Formmassen 0,01 bis 10, vorzugsweise 0,05 bis 5, und insbesondere 0,5 bis 2 Gew.-% eines
organischen Isocyanates R¹-N=C=O oder
Diisocyanates O=C=N-R²-N=C=O oder
deren Mischungen,
wobei der Rest R¹ der Komponente B) für lineare C1-C14-Alkylreste, verzweigte C3 bis C12-Alkylreste, unsubstituierte oder substituierte C3 bis C14-Cycloalkylreste, unsubstituierte oder substituierte aromatische Reste mit 6 bis 20 C-Atomen steht.

Unter linearen Alkylresten sollen unverzweigte Alkylketten mit 1 bis 14, vorzugsweise 1 bis 10 C-Atomen verstanden werden. Als Beispiele seien Methyl, Ethyl, n-Propyl, n-Butyl, n-Pentyl, n-Hexyl, n-Heptyl, n-Octyl, n-Nonyl und n-Decyl genannt.

Unter verzweigten Alkylresten sollen Alkylketten mit Verzweigungen verstanden werden, welche 3 bis 12, vorzugsweise 3 bis 10 C-Atome aufweisen.

Beispielhaft seien genannt: iso-propyl, 2-butyl, iso-butyl, tert-butyl, 1-methylbutyl, 2-methylbutyl, 3-methylbutyl, 2,2-dimethylpropyl, 1-ethylpropyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, 1-methylhexyl, 2-methylhexyl, 3-methylhexyl, 4-methylhexyl, 5-methylhexyl, 1-ethylpentyl, 2-ethylpentyl, 3-ethylpentyl, 1-methylheptyl, 2-methylheptyl, 3-methylheptyl, 4-methylheptyl, 5-methylheptyl, 1-propylpentyl, 1-ethylhexyl, 2-ethylhexyl, 3-ethylhexyl, 1-methyloctyl, 2-methylheptyl, 1-ethylhexyl, 2-ethylhexyl, 1,2-dimethylhexyl, 1-propylpentyl und 2-propylpentyl.

Als Cycloalkylreste mit 3 bis 14 C-Atomen, vorzugsweise 3 bis 10 C-Atomen seien beispielsweise Cyclopropyl, Cyclobutyl, Cyclopentyl, Cyclohexyl, Cycloheptyl, Cyclooctyl, Cyclononyl und Cyclodecyl genannt.

Unter substituierten Cycloalkylresten sollen insbesondere solche Reste verstanden werden, welche ein Heteroatom, vorzugsweise N oder O, im Ring aufweisen oder Substituenten wie einen oder mehrere Alkylreste mit 1 bis 4 C-Atomen tragen können.

Als Heterocyclen seien beispielsweise Tetrahydrofuran oder Pyrrolidin genannt.

Unter substituierten aromatischen Resten mit 6 bis 20, vorzugsweise mit 6 bis 17 C-Atomen sollen aromatische Ringsysteme wie Phenyl, Naphthyl, Anthracenyl oder Phenanthryl verstanden werden.

Derartige aromatische Reste können ein- oder mehrfach Substituenten tragen wie Alkylreste (linear oder verzweigt, siehe obige Definition) mit 1 bis 10, vorzugsweise 1 bis 4 C-Atomen oder Halogen, vorzugsweise Brom oder Chlor.

Ferner können die aromatischen Reste über Alkylenbrücken mit 1 bis 4 C-Atomen mit einem weiteren aromatischen Rest verbunden sein.

Als bevorzugte Verbindungen seien Cyclohexylisocyanat, Phenylisocyanat oder t-Butylisocyanat genannt.

Bevorzugte Reste R² sind lineare oder verzweigte C1 bis C14-Alkylenreste, unsubstituierte oder substituierte Cycloalkylenreste mit 3 bis 17 C-Atomen, substituierte oder unsubstituierte aromatische Reste mit 6 bis 20 C-Atomen.

Bevorzugte Alkylenreste weisen 1 bis 10 C-Atome auf. Beispielsweise seien Methylen, Ethylen, Propylen, Butylen, Pentamethylen, Hexamethylen und Heptamethylen genannt.

Beispiele für verzweigte Alkylenketten sind oben definierte Reste, welche einen oder mehrere Alkylreste mit 1 bis 4 C-Atomen tragen können.

Unsubstituierte Cycloalkylenreste weisen vorzugsweise 3 bis 14 C-Atome auf und entsprechen der obigen Definition für Cycloalkylreste, jedoch ist ein weiteres H-Atom durch eine Bindung ersetzt und bildet somit eine bivalente Einheit (oder bivalentes Radikal).

Als Beispiel seien Cyclohexylen, Cyclopentylen genannt.

Substituierte Cycloalkylenreste können Heteroatome wie N oder O im Ring aufweisen oder einen oder mehrere Alkylreste mit 1 bis 4 C-Atomen tragen. Weiterhin können derartige Reste über Alkylenbrücken mit 1 bis 4 C-Atomen mit einem weiteren Cycloalkylenrest verbunden sein, z.B. 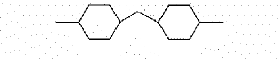 oder

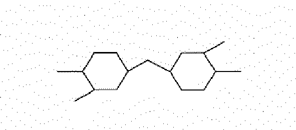

Unter substituierten oder unsubstituierten aromatischen Resten mit vorzugsweise 6 bis 17 C-Atomen werden o.g. Ringsysteme verstanden, in welchen ein weiteres H-Atom durch eine chemische Bindung ersetzt wurde und somit eine zweiwertige Einheit bildet (oder bivalentes Radikal genannt).

Im Einzelnen sind beispielhaft genannt: aliphatische Diisocyanate, wie Hexamethylen-diisocyanat, cycloaliphatische Diisocyanate, wie Isophoron-diisocyanat, 1,4-Cyclohexan-diisocyanat, 1-Methyl-2,4- und -2,6-cyclohexan-diisocyanat sowie die entsprechenden Isomerengemische, 4,4'-, 2,4'- und 2,2'-Dicyclohexylmethan-diisocyanat sowie die entsprechenden Isomerengemische und vorzugsweise aromatische Diisocyanate, wie 2,4-Toluylen-diisocyanat, Gemische aus 2,4- und 2,6-Toluylen-diisocyanat, 4,4'-, und 2,4'- und 2,2'-Diphenylmethan-diisocyanat, Gemische aus 2,4'- und 4,4'-Diphenylmethandiisocyanat, 4,4'-Diisocyanato-diphenylethan-(1,2) und 1,5-Naphthylen-diisocyanat. Vorzugsweise verwendet werden Hexamethylen-diisocyanat, Isophoron-diisocyanat, 1,5-Naphthylen-diisocyanat, Diphenylmethan-diisocyanat-Isomerengemische mit einem 4,4'-Diphenylmethan-diisocyanatgehalt von größer als 96 Gew.-% und insbesondere 4,4'-Diphenylmethan-diisocyanat.

Insbesondere bevorzugt sind:
trans-1,4-Cyclohexyldiisocyanat (CAS 7517-76-2)
Dicyclohexylmethan-4,4'-diisocyanat (CAS 5124-30-1)
4,4'-Methylenbis(phenylisocyanat) (CAS 101-68-8)
Toluol 2,4-diisocyanat (CAS 584-84-9)
Cyclohexylisocyanat (CAS 3173-53-3)
1,4-Phenylendiisocyanat (CAS 104-49-4)
Phenylisocyanat (CAS 103-71-9) sowie
Hexamethylendiisocyanat (CAS 822-06-0).

Als Komponente C) können die erfindungsgemäßen Formmassen 0 bis 60, vorzugsweise 0 bis 50 Gew.-% weiterer Zusatzstoffe enthalten.

Als Komponente C) können die Formmassen in Mengen 0 bis 40, bevorzugt 1 bis 30, insbesondere 2 bis 20 Gew.-% kautschukelastische Polymerisate (oft auch als Schlagzähmodifier, Elastomere oder Kautschuke bezeichnet) enthalten.

Ganz allgemein handelt es sich dabei um Copolymerisate die bevorzugt aus mindestens zwei der folgenden Monomeren aufgebaut sind: Ethylen, Propylen, Butadien, Isobuten, Isopren, Chloropren, Vinylacetat, Styrol, Acrylnitril und Acryl- bzw. Methacrylsäureester mit 1 bis 18 C-Atomen in der Alkoholkomponente.

Derartige Polymere werden z.B. in Houben-Weyl, Methoden der organischen Chemie, Bd. 14/1 (Georg-Thieme-Verlag, Stuttgart, 1961). Seiten 392 bis 406 und in der Monographie von C.B. Bucknall, "Toughened Plastics" (Applied Science Publishers, London, 1977) beschrieben.

Im Folgenden werden einige bevorzugte Arten solcher Elastomerer vorgestellt.

Bevorzugte Arten von solchen Elastomeren sind die sog. Ethylen-Propylen (EPM) bzw. Ethylen-Propylen-Dien-(EPDM)-Kautschuke.

EPM-Kautschuke haben im Allgemeinen praktisch keine Doppelbindungen mehr, während EPDM-Kautschuke 1 bis 20 Doppelbindungen/100 C-Atome aufweisen können.

Als Dien-Monomere für EPDM-Kautschuke seien beispielsweise konjugierte Diene wie Isopren und Butadien, nicht-konjugierte Diene mit 5 bis 25 C-Atomen wie Penta-1,4-dien, Hexa-1,4-dien, Hexa-1,5-dien, 2,5-Dimethylhexa-1,5-dien und Octa-1,4-dien, cyclische Diene wie Cyclopentadien, Cyclohexadiene, Cyclooctadiene und Dicyclopen-tadien sowie Alkenylnorbornene wie 5-Ethyliden-2-norbornen, 5-Butyliden-2-norbornen, 2-Methallyl-5-norbornen, 2-Isopropenyl-5-norbornen und Tricyclodiene wie 3-Methyl-tricyclo(5.2.1.0.2.6)-3,8-decadien oder deren Mischungen genannt. Bevorzugt werden Hexa-1,5-dien, 5-Ethylidennorbornen und Dicyclopentadien. Der Diengehalt der EPDM-Kautschuke beträgt vorzugsweise 0,5 bis 50, insbesondere 1 bis 8 Gew.-%, bezogen auf das Gesamtgewicht des Kautschuks.

EPM- bzw. EPDM-Kautschuke können vorzugsweise auch mit reaktiven Carbonsäuren oder deren Derivaten gepfropft sein. Hier seien z.B. Acrylsäure, Methacrylsäure und deren Derivate, z.B. Glycidyl(meth)acrylat, sowie Maleinsäureanhydrid genannt.

Eine weitere Gruppe bevorzugter Kautschuke sind Copolymere des Ethylens mit Acrylsäure und/oder Methacrylsäure und/oder den Estern dieser Säuren. Zusätzlich können die Kautschuke noch Dicarbonsäuren wie Maleinsäure und Fumarsäure oder Derivate dieser Säuren, z.B. Ester und Anhydride, und/oder Epoxy-Gruppen enthaltende Monomere enthalten. Diese Dicarbonsäurederivate bzw. Epoxygruppen enthaltende Monomere werden vorzugsweise durch Zugabe von Dicarbonsäure- bzw. Epoxygruppen enthaltenden Monomeren der allgemeinen Formeln I oder II oder III oder IV zum Monomerengemisch in den Kautschuk eingebaut

R¹C(COOR²)=C(COOR³)R⁴ (I)

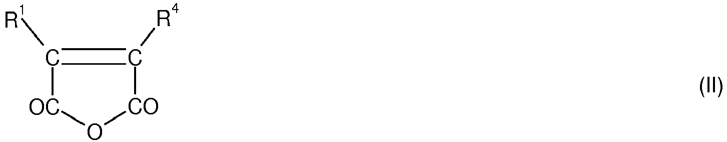

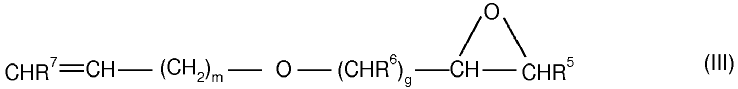

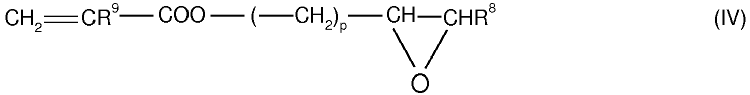

wobei R¹ bis R⁹ Wasserstoff oder Alkylgruppen mit 1 bis 6 C-Atomen darstellen und m eine ganze Zahl von 0 bis 20, g eine ganze Zahl von 0 bis 10 und p eine ganze Zahl von 0 bis 5 ist. Vorzugsweise bedeuten die Reste R¹ bis R⁹ Wasserstoff, wobei m für 0 oder 1 und g für 1 steht. Die entsprechenden Verbindungen sind Maleinsäure, Fumarsäure, Maleinsäureanhydrid, Allylglycidylether und Vinylglycidylether.

Bevorzugte Verbindungen der Formeln I, II und IV sind Maleinsäure, Maleinsäureanhydrid und Epoxygruppen-enthaltende Ester der Acrylsäure und/oder Methacrylsäure, wie Glycidylacrylat, Glycidylmethacrylat und die Ester mit tertiären Alkoholen, wie t-Butylacrylat. Letztere weisen zwar keine freien Carboxylgruppen auf, kommen in ihrem Verhalten aber den freien Säuren nahe und werden deshalb als Monomere mit latenten Carboxylgruppen bezeichnet.
Vorteilhaft bestehen die Copolymeren aus 50 bis 98 Gew.-% Ethylen, 0,1 bis 20 Gew.-% Epoxygruppen enthaltenden Monomeren und/oder Methacrylsäure und/oder Säureanhydridgruppen enthaltenden Monomeren sowie der restlichen Menge an (Meth)acrylsäureestern.

Besonders bevorzugt sind Copolymerisate aus
- 50 bis 98,: insbesondere 55 bis 95 Gew.-% Ethylen,
- 0,1 bis 40,: insbesondere 0,3 bis 20 Gew.-% Glycidylacrylat und/oder Glycidyl-methacrylat, (Meth)acrylsäure und/oder Maleinsäureanhydrid, und
- 1 bis 45,: insbesondere 5 bis 40 Gew.-% n-Butylacrylat und/oder 2-Ethylhexylacrylat.

Weitere bevorzugte Ester der Acryl- und/oder Methacrylsäure sind die Methyl-, Ethyl-, Propyl- und i- bzw. t-Butylester.

Daneben können auch Vinylester und Vinylether als Comonomere eingesetzt werden.

Die vorstehend beschriebenen Ethylencopolymeren können nach an sich bekannten Verfahren hergestellt werden, vorzugsweise durch statistische Copolymerisation unter hohem Druck und erhöhter Temperatur. Entsprechende Verfahren sind allgemein bekannt.

Bevorzugte Elastomere sind auch Emulsionspolymerisate, deren Herstellung z.B. bei Blackley in der Monographie "Emulsion Polymerization" beschrieben wird. Die verwendbaren Emulgatoren und Katalysatoren sind an sich bekannt.

Grundsätzlich können homogen aufgebaute Elastomere oder aber solche mit einem Schalenaufbau eingesetzt werden. Der schalenartige Aufbau wird durch die Zugabereihenfolge der einzelnen Monomeren bestimmt; auch die Morphologie der Polymeren wird von dieser Zugabereihenfolge beeinflusst.

Nur stellvertretend seien hier als Monomere für die Herstellung des Kautschukteils der Elastomeren Acrylate wie z.B. n-Butylacrylat und 2-Ethylhexylacrylat, entsprechende Methacrylate, Butadien und Isopren sowie deren Mischungen genannt. Diese Monomeren können mit weiteren Monomeren wie z.B. Styrol, Acrylnitril, Vinylethern und weiteren Acrylaten oder Methacrylaten wie Methylmethacrylat, Methylacrylat, Ethylacrylat und Propylacrylat copolymerisiert werden.

Die Weich- oder Kautschukphase (mit einer Glasübergangstemperatur von unter 0°C) der Elastomeren kann den Kern, die äußere Hülle oder eine mittlere Schale (bei Elastomeren mit mehr als zweischaligem Aufbau) darstellen; bei mehrschaligen Elastomeren können auch mehrere Schalen aus einer Kautschukphase bestehen.
Sind neben der Kautschukphase noch eine oder mehrere Hartkomponenten (mit Glasübergangstemperaturen von mehr als 20°C) am Aufbau des Elastomeren beteiligt, so werden diese im allgemeinen durch Polymerisation von Styrol, Acrylnitril, Methacrylnitril, α-Methylstyrol, p-Methylstyrol, Acrylsäureestern und Methacrylsäureestern wie Methylacrylat, Ethylacrylat und Methylmethacrylat als Hauptmonomeren hergestellt. Daneben können auch hier geringere Anteile an weiteren Comonomeren eingesetzt werden.

In einigen Fällen hat es sich als vorteilhaft herausgestellt, Emulsionspolymerisate einzusetzen, die an der Oberfläche reaktive Gruppen aufweisen. Derartige Gruppen sind z.B. Epoxy-, Carboxyl-, latente Carboxyl-, Amino- oder Amidgruppen sowie funktionelle Gruppen, die durch Mitverwendung von Monomeren der allgemeinen Formel 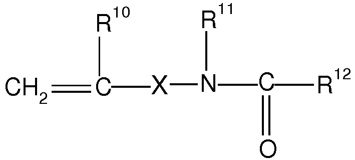 eingeführt werden können,
wobei die Substituenten folgende Bedeutung haben können:
- R¹⁰: Wasserstoff oder eine C₁- bis C₄-Alkylgruppe,
- R¹¹: Wasserstoff, eine C₁- bis C₈-Alkylgruppe oder eine Arylgruppe, insbesondere Phenyl,
- R¹²: Wasserstoff, eine C₁- bis C₁₀-Alkyl-, eine C₆- bis C₁₂-Arylgruppe oder -OR¹³
- R¹³: eine C₁- bis C₈-Alkyl- oder C₆- bis C₁₂-Arylgruppe, die gegebenenfalls mit O- oder N-haltigen Gruppen substituiert sein können,
- X: eine chemische Bindung, eine C₁- bis C₁₀-Alkylen- oder C₆-C₁₂-Arylengruppe oder 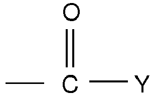
- Y: O-Z oder NH-Z und
- Z eine: C₁- bis C₁₀-Alkylen- oder C₆- bis C₁₂-Arylengruppe.

Auch die in der EP-A208 187 beschriebenen Pfropfmonomeren sind zur Einführung reaktiver Gruppen an der Oberfläche geeignet.
Als weitere Beispiele seien noch Acrylamid, Methacrylamid und substituierte Ester der Acrylsäure oder Methacrylsäure wie (N-t-Butylamino)-ethylmethacrylat, (N,N-Dimethylamino)ethylacrylat, (N,N-Dimethylamino)-methylacrylat und (N,N-Diethylamino)ethylacrylat genannt.

Weiterhin können die Teilchen der Kautschukphase auch vernetzt sein. Als Vernetzer wirkende Monomere sind beispielsweise Buta-1,3-dien, Divinylbenzol, Diallylphthalat und Dihydrodicyclopentadienylacrylat sowie die in der EP-A 50 265 beschriebenen Verbindungen.

Ferner können auch sogenannte pfropfvernetzende Monomere (graft-linking monomers) verwendet werden, d.h. Monomere mit zwei oder mehr polymerisierbaren Doppelbindungen, die bei der Polymerisation mit unterschiedlichen Geschwindigkeiten reagieren. Vorzugsweise werden solche Verbindungen verwendet, in denen mindestens eine reaktive Gruppe mit etwa gleicher Geschwindigkeit wie die übrigen Monomeren polymerisiert, während die andere reaktive Gruppe (oder reaktive Gruppen) z.B. deutlich langsamer polymerisiert (polymerisieren). Die unterschiedlichen Polymerisationsgeschwindigkeiten bringen einen bestimmten Anteil an ungesättigten Doppelbindungen im Kautschuk mit sich. Wird anschließend auf einen solchen Kautschuk eine weitere Phase aufgepfropft, so reagieren die im Kautschuk vorhandenen Doppelbindungen zumindest teilweise mit den Pfropfmonomeren unter Ausbildung von chemischen Bindungen, d.h. die aufgepfropfte Phase ist zumindest teilweise über chemische Bindungen mit der Pfropfgrundlage verknüpft.

Beispiele für solche pfropfvernetzende Monomere sind Allylgruppen enthaltende Monomere, insbesondere Allylester von ethylenisch ungesättigten Carbonsäuren wie Allyl-acrylat, Allylmethacrylat, Diallylmaleat, Diallylfumarat, Diallylitaconat oder die entsprechenden Monoallylverbindungen dieser Dicarbonsäuren. Daneben gibt es eine Vielzahl weiterer geeigneter pfropfvernetzender Monomerer; für nähere Einzelheiten sei hier beispielsweise auf die US-PS 4 148 846 verwiesen.

Im Allgemeinen beträgt der Anteil dieser vernetzenden Monomeren an dem schlagzäh modifizierenden Polymer bis zu 5 Gew.-%, vorzugsweise nicht mehr als 3 Gew.-%, bezogen auf das schlagzäh modifizierende Polymere.

Nachfolgend seien einige bevorzugte Emulsionspolymerisate aufgeführt. Zunächst sind hier Pfropfpolymerisate mit einem Kern und mindestens einer äußeren Schale zu nennen, die folgenden Aufbau haben:

| Typ | Monomere für den Kern | Monomere für die Hülle |
|---|---|---|
| I | Buta-1,3-dien, Isopren, n-Butylacrylat, Ethylhexylacrylat oder deren Mischungen | Styrol, Acrylnitril, Methylmethacrylat |
| II | wie I aber unter Mitverwendung von Vernetzern | wie I |
| III | wie I oder II | n-Butylacrylat, Ethylacrylat, Methylacrylat, Buta-1,3-dien, Isopren, Ethylhexyl-acrylat |
| IV | wie I oder II | wie I oder III aber unter Mitverwendung von Monomeren mit reaktiven Gruppen wie hierin beschrieben |
| V | Styrol, Acrylnitril, Methylmethacrylat oder deren Mischungen | erste Hülle aus Monomeren wie unter I und II für den Kern beschrieben zweite Hülle wie unter I oder IV für die Hülle beschrieben |

Anstelle von Pfropfpolymerisaten mit einem mehrschaligen Aufbau können auch homogene, d.h. einschalige Elastomere aus Buta-1,3-dien, Isopren und n-Butylacrylat oder deren Copolymeren eingesetzt werden. Auch diese Produkte können durch Mitverwendung von vernetzenden Monomeren oder Monomeren mit reaktiven Gruppen hergestellt werden.

Beispiele für bevorzugte Emulsionspolymerisate sind n-Butylacrylat/(Meth)acrylsäure-Copolymere, n-Butylacrylat/Glycidylacrylat- oder n-Butylacrylat/Glycidylmethacrylat-Copolymere, Pfropfpolymerisate mit einem inneren Kern aus n-Butylacrylat oder auf Butadienbasis und einer äußeren Hülle aus den vorstehend genannten Copolymeren und Copolymere von Ethylen mit Comonomeren, die reaktive Gruppen liefern.

Die beschriebenen Elastomere können auch nach anderen üblichen Verfahren, z.B. durch Suspensionspolymerisation, hergestellt werden.

Siliconkautschuke, wie in der DE-A 37 25 576, der EP-A 235 690, der DE-A 38 00 603 und der EP-A 319 290 beschrieben, sind ebenfalls bevorzugt.

Besonders bevorzugte Kautschuke C) sind Ethylencopolymere, wie vorstehend beschrieben, welche funktionelle Monomere enthalten, wobei die funktionellen Monomeren ausgewählt sind aus der Gruppe der Carbonsäure-, Carbonsäureanhydrid-, Carbonsäureester-, Carbonsäureamid-, Carbonsäureimid-, Amino-, Hydroxyl-, Epoxid-, Urethan- oder Oxazolingruppen oder deren Mischungen.

Der Anteil der funktionellen Gruppen beträgt 0,1 bis 20, vorzugsweise 0,2 bis 10 und insbesondere 0,3 bis 7 Gew.-%, bezogen auf 100 Gew.-% C).

Besonders bevorzugte Monomere sind aus einer ethylenisch ungesättigten Mono- oder Dicarbonsäure oder einem funktionellen Derivat einer solchen Säure aufgebaut.

Grundsätzlich eignen sich alle primären, sekundären und tertiären C₁-C₁₈-Alkylester der Acrylsäure oder Methacrylsäure, doch werden Ester mit 1 - 12 C-Atomen, insbesondere mit 2 - 10 C-Atomen bevorzugt.

Beispiele hierfür sind Methyl-, Ethyl-, Propyl-, n-, i-Butyl- und t-Butyl-, 2-Ethylhexyl-, Octyl- und Decylacrylate bzw. die entsprechenden Ester der Methacrylsäure. Von diesen werden n-Butylacrylat und 2-Ethylhexylacrylat besonders bevorzugt.

Anstelle der Ester oder zusätzlich zu diesen können in den Olefinpolymerisaten auch säurefunktionelle und/oder latent säurefunktionelle Monomere ethylenisch ungesättigter Mono- oder Dicarbonsäuren oder Epoxygruppen aufweisende Monomere enthalten sein.

Als weitere Beispiele für Monomere seien Acrylsäure, Methacrylsäure, tertiäre Alkylester dieser Säuren, insbesondere tert.-Butylacrylat und Dicarbonsäuren wie Maleinsäure und Fumarsäure oder Derivate dieser Säuren sowie deren Monoester genannt.

Als latent säurefunktionelle Monomere sollen solche Verbindungen verstanden werden, die unter den Polymerisationsbedingungen bzw. bei der Einarbeitung der Olefinpolymerisate in die Formmassen freie Säuregruppen bilden. Als Beispiele hierfür seien Anhydride von Dicarbonsäuren mit bis zu 20 C-Atomen, insbesondere Maleinsäureanhydrid und tertiäre C₁-C₁₂-Alkylester der vorstehend genannten Säuren, insbesondere tert.-Butylacrylat und tert.-Butylmethacrylat angeführt.

Die säurefunktionellen bzw. latent säurefunktionellen Monomeren und die Epoxygruppen-enthaltenden Monomeren werden vorzugsweise durch Zugabe von Verbindungen der allgemeinen Formeln I - IV zum Monomerengemisch in die Olefinpolymerisate eingebaut.

Der Schmelzindex der Ethylencopolymeren liegt im Allgemeinen im Bereich von 1 bis 80 g/10 min (gemessen bei 190°C und 2,16 kg Belastung).

Das Molekulargewicht dieser Ethylen-α-Olefin-Copolymere liegt zwischen 10.000 und 500.000 g/mol, bevorzugt zwischen 15.000 und 400.000 g/mol (Mn, bestimmt mittels GPC in 1,2,4-Trichlorbenzol mit PS-Eichung).

In einer besonderen Ausführungsform werden mittels sog. "single site catalysts" hergestellte Ethylen-α-Olefin-Copolymere eingesetzt. Weitere Einzelheiten können der US 5,272,236 entnommen werden. In diesem Fall weisen die Ethylen-α-Olefin-Copolymere eine für Polyolefine enge Molekulargewichtsverteilung kleiner 4, vorzugsweise kleiner 3,5 auf.

Bevorzugt eingesetzte Handelsprodukte sind Exxelor® VA 1801 oder 1803, Kraton® G 1901 FX oder Fusabond® N NM493 D oder Fusabond® A560 der Firmen Exxon, Kraton und DuPont sowie Tafmer®MH 7010 der Firma Mitsui.

Selbstverständlich können auch Mischungen der vorstehend aufgeführten Kautschuktypen eingesetzt werden.

Als Komponente C) können die erfindungsgemäßen Formmassen bis zu 60, vorzugsweise bis zu 50 Gew.-% weiterer Zusatzstoffe enthalten.

Als faser- oder teilchenförmige Füllstoffe C) seien Kohlenstofffasern, Glasfasern, Glaskugeln, amorphe Kieselsäure, Calciumsilicat, Calciummetasilicat, Magnesiumcarbonat, Kaolin, Kreide, gepulverter Quarz, Glimmer, Bariumsulfat und Feldspat genannt, die in Mengen von 1 bis 50 Gew.-%, insbesondere 5 bis 40, vorzugsweise 10 bis 40 Gew.-% eingesetzt werden.

Als bevorzugte faserförmige Füllstoffe seien Kohlenstofffasern, Aramid-Fasern und Kaliumtitanat-Fasern genannt, wobei Glasfasern als E-Glas besonders bevorzugt sind. Diese können als Rovings oder Schnittglas in den handelsüblichen Formen eingesetzt werden.

Die faserförmigen Füllstoffe können zur besseren Verträglichkeit mit den Thermoplasten mit einer Silanverbindung oberflächlich vorbehandelt sein.

Geeignete Silanverbindungen sind solche der allgemeinen Formel

(X-(CH₂)ₙ)ₖ-Si-(O-CₘH₂ₘ₊₁)₄₋ₖ

in der die Substituenten folgende Bedeutung haben: X NH₂-, 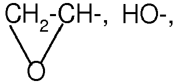
- n: eine ganze Zahl von 2 bis 10, bevorzugt 3 bis 4
- m: eine ganze Zahl von 1 bis 5, bevorzugt 1 bis 2
- k: eine ganze Zahl von 1 bis 3, bevorzugt 1

Bevorzugte Silanverbindungen sind Aminopropyltrimethoxysilan, Aminobutyltrimeth-oxysilan, Aminopropyltriethoxysilan, Aminobutyltriethoxysilan sowie die entsprechenden Silane, welche als Substituent X eine Glycidylgruppe enthalten.

Die Silanverbindungen werden im Allgemeinen in Mengen von 0,01 bis 2, vorzugsweise 0,025 bis 1,0 und insbesondere 0,05 bis 0,5 Gew.-% (bezogen auf C)) zur Oberflächenbeschichtung eingesetzt.

Geeignet sind auch nadelförmige mineralische Füllstoffe.

Unter nadelförmigen mineralischen Füllstoffen wird im Sinne der Erfindung ein mineralischer Füllstoff mit stark ausgeprägtem nadelförmigen Charakter verstanden. Als Beispiel sei nadelförmiger Wollastonit genannt. Vorzugsweise weist das Mineral ein L/D-(Länge Durchmesser-Verhältnis von 8 : 1 bis 35 : 1, bevorzugt von 8 : 1 bis 11 : 1 auf. Der mineralische Füllstoff kann gegebenenfalls mit den vorstehend genannten Silanverbindungen vorbehandelt sein; die Vorbehandlung ist jedoch nicht unbedingt erforderlich.

Als weitere Füllstoffe seien Kaolin, calciniertes Kaolin, Wollastonit, Talkum und Kreide genannt sowie zusätzlich plättchen- oder nadelförmige Nanofüllstoffe bevorzugt in Mengen zwischen 0,1 und 10 %. Bevorzugt werden hierfür Böhmit, Bentonit, Montmorillonit, Vermicullit und Hektorit eingesetzt. Um eine gute Verträglichkeit der plättchenförmigen Nanofüllstoffe mit dem organischen Bindemittel zu erhalten, werden die plättchenförmigen Nanofüllstoffe nach dem Stand der Technik organisch modifiziert. Der Zusatz der plättchen- oder nadelförmigen Nanofüllstoffe zu den erfindungsgemäßen Nanokompositen führt zu einer weiteren Steigerung der mechanischen Festigkeit.

Als Komponente C) können die erfindungsgemäßen Formmassen 0,05 bis 3, vorzugsweise 0,1 bis 1,5 und insbesondere 0,1 bis 1 Gew.-% eines Schmiermittels enthalten.

Bevorzugt sind Al-, Alkali-, Erdalkalisalze oder Ester- oder Amide von Fettsäuren mit 10 bis 44 C-Atomen, vorzugsweise mit 12 bis 44 C-Atomen.

Die Metallionen sind vorzugsweise Erdalkali und Al, wobei Ca oder Mg besonders bevorzugt sind.

Bevorzugte Metallsalze sind Ca-Stearat und Ca-Montanat sowie Al-Stearat.

Es können auch Mischungen verschiedener Salze eingesetzt werden, wobei das Mischungsverhältnis beliebig ist.

Die Carbonsäuren können 1- oder 2-wertig sein. Als Beispiele seien Pelargonsäure, Palmitinsäure, Laurinsäure, Margarinsäure, Dodecandisäure, Behensäure und besonders bevorzugt Stearinsäure, Caprinsäure sowie Montansäure (Mischung von Fettsäuren mit 30 bis 40 C-Atomen) genannt.

Die aliphatischen Alkohole können 1- bis 4-wertig sein. Beispiele für Alkohole sind n-Butanol, n-Octanol, Stearylalkohol, Ethylenglykol, Propylenglykol, Neopentylglykol, Pentaerythrit, wobei Glycerin und Pentaerythrit bevorzugt sind.

Die aliphatischen Amine können 1- bis 3-wertig sein. Beispiele hierfür sind Stearylamin, Ethylendiamin, Propylendiamin, Hexamethylendiamin, Di(6-Aminohexyl)amin, wobei Ethylendiamin und Hexamethylendiamin besonders bevorzugt sind. Bevorzugte Ester oder Amide sind entsprechend Glycerindistearat, Glycerintristearat, Ethylendiamindistearat, Glycerinmonopalmitat, Glycerintrilaurat, Glycerinmonobehenat und Penta-erythrittetrastearat.

Es können auch Mischungen verschiedener Ester oder Amide oder Ester mit Amiden in Kombination eingesetzt werden, wobei das Mischungsverhältnis beliebig ist.

Als sterisch gehinderte Phenole C) eignen sich prinzipiell alle Verbindungen mit phenolischer Struktur, die am phenolischen Ring mindestens eine sterisch anspruchsvolle Gruppe aufweisen.

Vorzugsweise kommen z.B. Verbindungen der Formel 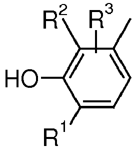 in Betracht, in der bedeuten:
R¹ und R² eine Alkylgruppe, eine substituierte Alkylgruppe oder eine substituierte Triazolgruppe, wobei die Reste R¹ und R² gleich oder verschieden sein können und R³ eine Alkylgruppe, eine substituierte Alkylgruppe, eine Alkoxigruppe oder eine substituierte Aminogruppe.

Antioxidantien der genannten Art werden beispielsweise in der DE-A 27 02 661 (US 4 360 617) beschrieben.

Eine weitere Gruppe bevorzugter sterisch gehinderter Phenole leiten sich von substituierten Benzolcarbonsäuren ab, insbesondere von substituierten Benzolpropionsäuren.

Besonders bevorzugte Verbindungen aus dieser Klasse sind Verbindungen der Formel 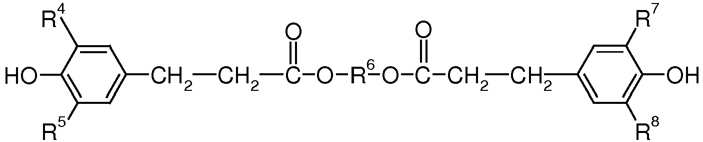 wobei R⁴, R⁵, R⁷ und R⁸ unabhängig voneinander C₁-C₈-Alkylgruppen darstellen, die ihrerseits substituiert sein können (mindestens eine davon ist eine sterisch anspruchsvolle Gruppe) und R⁶ einen zweiwertigen aliphatischen Rest mit 1 bis 10 C-Atomen bedeutet, der in der Hauptkette auch C-O-Bindungen aufweisen kann.

Bevorzugte Verbindungen, die dieser Formel entsprechen, sind 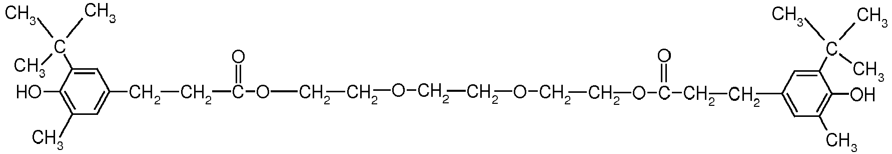 (Irganox® 245 der Firma BASF SE) 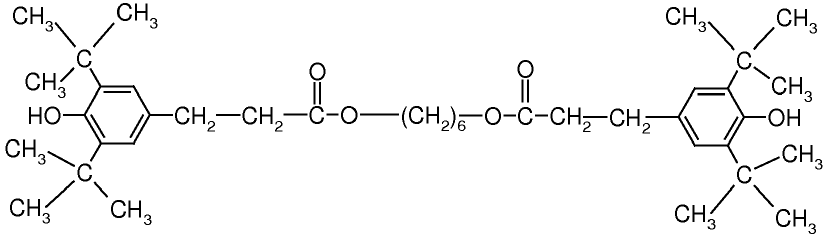 (Irganox® 259 der Firma BASF SE)

Beispielhaft genannt seien insgesamt als sterisch gehinderte Phenole:
2,2'-Methylen-bis-(4-methyl-6-tert.-butylphenol), 1,6-Hexandiol-bis[3-(3,5-di-tert.-butyl-4-hydroxyphenyl)-propionat], Pentaerythrityl-tetrakis-[3-(3,5-di-tert.-butyl-4-hydroxy-phenyl)-propionat], Distearyl-3,5-di-tert.-butyl-4-hydroxybenzylphosphonat, 2,6,7-Trioxa-1-phosphabicyclo-[2.2.2]oct-4-yl-methyl-3,5-di-tert.-butyl-4-hydroxyhydro-cinnamat, 3,5-Di-tert.-butyl-4-hydroxyphenyl-3,5-distearyl-thiotriazylamin, 2-(2'-Hydroxy-3'-hydroxy-3',5'-di-tert.-butylphenyl)-5-chlorbenzotriazol, 2,6-Di-tert.-butyl-4-hydroxymethylphenol, 1,3,5-Trimethyl-2,4,6-tris-(3,5-di-tert.-butyl-4-hydroxybenzyl)-benzol, 4,4'-Methylen-bis-(2,6-di-tert.-butylphenol), 3,5-Di-tert.-butyl-4-hydroxybenzyl-dimethylamin.

Als besonders wirksam erwiesen haben sich und daher vorzugsweise verwendet werden 2,2'-Methylen-bis-(4-methyl-6-tert.-butylphenol), 1,6-Hexandiol-bis-(3,5-di-tert.-butyl-4-hydroxyphenyl)-propionat (Irganox® 259), Pentaerythrityl-tetrakis-[3-(3,5-di-tert.-butyl-4-hydroxyphenyl)-propionat] sowie N,N'-Hexamethylen-bis-3,5-di-tert.-butyl-4-hydroxyhydrocinnamid (Irganox® 1098) und das vorstehend beschriebene Irganox® 245 der Firma BASF SE, das besonders gut geeignet ist.
Die Antioxidantien C), die einzeln oder als Gemische eingesetzt werden können, sind in einer Menge von 0,05 bis zu 3 Gew.-%, vorzugsweise von 0,1 bis 1,5 Gew.-%, insbesondere 0,1 bis 1 Gew.-%, bezogen auf das Gesamtgewicht der Formmassen A) bis C) enthalten.

In manchen Fällen haben sich sterisch gehinderte Phenole mit nicht mehr als einer sterisch gehinderten Gruppe in ortho-Stellung zur phenolischen Hydroxygruppe als besonders vorteilhaft erwiesen; insbesondere bei der Beurteilung der Farbstabilität bei Lagerung in diffusem Licht über längere Zeiträume.

Als Komponente C) können die erfindungsgemäßen Formmassen 0,05 bis 5, vorzugsweise 0,1 bis 2 und insbesondere 0,25 bis 1,5 Gew.-% eines Nigrosins enthalten.

Unter Nigrosinen versteht man im allgemeinen eine Gruppe von schwarzen oder grauen, mit den Indulinen verwandten Phenazin-Farbstoffen (Azin-Farbstoffen) in verschiedenen Ausführungsformen (wasserlöslich, fettlöslich, spritlöslich), die bei Wollfärberei und -druck, beim Schwarzfärben von Seiden, zum Färben von Leder, Schuhcremes, Firnissen, Kunststoffen, Einbrennlacken, Tinten und dergleichen, sowie als Mikroskopiefarbstoffe Verwendung finden.

Man gewinnt die Nigrosine technisch durch Erhitzen von Nitrobenzol, Anilin und salzsaurem Anilin mit Metall. Eisen und FeCl₃ (Name von lateinischem niger = schwarz).

Die Komponente C) kann als freie Base oder auch als Salz (z.B. Hydrochlorid) eingesetzt werden.

Weitere Einzelheiten zu Nigrosinen sind beispielsweise dem elektronischen Lexikon Römpp Online, Version 2.8, Thieme-Verlag Stuttgart, 2006, Stichwort "Nigrosin" zu entnehmen.

Als Komponente C) können die erfindungsgemäßen Formmassen 0 bis 20, vorzugsweise 1 bis 15 und insbesondere 5 bis 15 Gew.-% roten Phosphor oder/und eines stickstoffhaltigen Flammschutzmittels, vorzugsweise einer Melaminverbindung enthalten.

Geeignete Verbindungen (oft auch als Salze oder Addukte bezeichnet) sind Melaminsulfat, Melamin, Melaminborat, -oxalat, -phosphat prim., -phosphat sec. und -pyrophosphat sec., Neopentylglycolborsäuremelamin sowie polymeres Melaminphosphat (CAS-Nr 56386-64-2 bzw. 218768-84-4).

Als Komponente C) können die erfindungsgemäßen thermoplastischen Formmassen übliche Verarbeitungshilfsmittel wie Stabilisatoren, Oxidationsverzögerer, Mittel gegen Wärmezersetzung und Zersetzung durch ultraviolettes Licht, Gleit- und Entformungsmittel, Färbemittel wie Farbstoffe und Pigmente, Keimbildungsmittel, Weichmacher, usw. enthalten.

Als Beispiele für Oxidationsverzögerer und Wärmestabilisatoren sind sterisch gehinderte Phenole und/oder Phosphite und Amine (z.B. TAD), Hydrochinone, aromatische sekundäre Amine wie Diphenylamine, verschiedene substituierte Vertreter dieser Gruppen und deren Mischungen in Konzentrationen bis zu 1 Gew.-%, bezogen auf das Gewicht der thermoplastischen Formmassen genannt.

Als UV-Stabilisatoren, die im Allgemeinen in Mengen bis zu 2 Gew.-%, bezogen auf die Formmasse, verwendet werden, seien verschiedene substituierte Resorcine, Salicylate, Benzotriazole und Benzophenone genannt.

Es können anorganische Pigmente, wie Titandioxid, Ultramarinblau, Eisenoxid und Ruß, weiterhin organische Pigmente, wie Phthalocyanine, Chinacridone, Perylene sowie Farbstoffe, wie Anthrachinone als Farbmittel zugesetzt werden.

Als Keimbildungsmittel können Natriumphenylphosphinat, Aluminiumoxid, Siliziumdioxid sowie bevorzugt Talkum eingesetzt werden.

Die erfindungsgemäßen thermoplastischen Formmassen können nach an sich bekannten Verfahren hergestellt werden, indem man die Ausgangskomponenten in üblichen Mischvorrichtungen wie Schneckenextrudern, Brabender-Mühlen oder Banbury-Mühlen mischt und anschließend extrudiert. Nach der Extrusion kann das Extrudat abgekühlt und zerkleinert werden. Es können auch einzelne Komponenten vorgemischt werden und dann die restlichen Ausgangsstoffe einzeln und/oder ebenfalls gemischt hinzugegeben werden. Die Mischtemperaturen liegen in der Regel bei 230 bis 320°C.

Nach einer weiteren bevorzugten Arbeitsweise können die Komponenten B) sowie gegebenenfalls C) mit einem Präpolymeren gemischt, konfektioniert und granuliert werden. Das erhaltene Granulat wird in fester Phase anschließend unter Inertgas kontinuierlich oder diskontinuierlich bei einer Temperatur unterhalb des Schmelzpunktes der Komponente A) bis zur gewünschten Viskosität kondensiert.

Die erfindungsgemäß verwendbaren Formmassen eignen sich zur Herstellung von Formkörpern jeglicher Art, welche eine verbesserte (Laser)Transparenz und/oder verminderte Trübung aufweisen. Diese Formmassen weisen mindestens eine der folgenden Vorteile auf:
- Der Haze-Wert ist um mindestens 5% niedriger verglichen mit einer Referenzpolymerzusammensetzung ohne die Komponente B), gemessen nach ASTM D1003 (bei einer Probenkörperdicke von 1,3 mm);
- Der Clarity-Wert ist um mindestens 5% höher verglichen mit einer Referenzpolymerzusammensetzung ohne die Komponente B), gemessen nach ASTM D1003 (bei einer Probenkörperdicke von 1,3 mm);
- Die Lasertransparenz ist um mindestens 1% höher verglichen mit einer Referenzpolymerzusammensetzung ohne die Komponente B), gemessen bei 1064 nm (bei einer Probenkörperdicke von 1,3 mm).

Der hier verwendete Begriff "Haze" ist definiert als der Prozentsatz an transmittiertem Licht welcher durch das durchqueren eines Probekörpers (Platte) im Durchschnitt um mehr als 2.5° vom einfallenden Licht abweicht. Der Haze wird nach ASTM D1003 bestimmt. Die erfindungsgemäß verwendbaren Formmassen weisen einen Haze auf, welcher mindestens 5% niedriger, bevorzugt 10% niedriger, besonders bevorzugt 15% niedriger und insbesondere 20% niedriger liegt verglichen mit einer Referenzpolymerzusammensetzung ohne die Komponente B), gemessen bei einer Probenkörperdicke (Platte) von 1,3 mm.

Der hier verwendete Begriff "Clarity" ist definiert als der Prozentsatz an transmittiertem Licht welcher durch das durchqueren eines Probekörpers (Platte) im Durchschnitt um weniger als 2,5° vom einfallenden Licht abweicht. Die Clarity wird nach ASTM D1003 bestimmt. Die erfindungsgemäß verwendbaren Formmassen weisen eine Clarity auf, welche mindestens 5% höher, bevorzugt 10% höher, besonders bevorzugt 15% höher und insbesondere 20% höher liegt verglichen mit einer Referenzpolymerzusammensetzung ohne die Komponente B), gemessen bei einer Probenkörperdicke (Platte) von 1,3 mm.

Die erfindungsgemäß verwendbaren Formmassen weisen eine Lasertransparenz auf, welche mindestens 1% höher, bevorzugt 3% höher, besonders bevorzugt 5% höher und insbesondere 10% höher liegt verglichen mit einer Referenzpolymerzusammensetzung ohne die Komponente B), gemessen bei einer Probenkörperdicke (Platte) von 1,3 mm.

Die Bestimmung der Lasertransparenz bei einer Wellenlänge von 1064 nm wurde mittels einer thermoelektrischen Leistungsmessung durchgeführt. Die Messgeometrie stellte sich wie folgt dar:
Von einem Laserstrahl (diodengepumpter Nd-YAG-Laser mit einer Wellenlänge von 1064 nm, FOBA DP50) mit einer Gesamtleistung von 2 Watt wurde mittels eines Strahlteilers (Typ SQ2 nichtpolarisierender Strahlteiler von Fa. Laseroptik GmbH) ein Referenzstrahl im Winkel 90° mit 1 Watt Leistung abgeteilt. Dieser traf auf den Referenzsensor. Der den Strahlteiler passierende Teil des ursprünglichen Strahls stellte den Messstrahl mit ebenfalls 1 Watt Leistung dar. Dieser wurde durch eine Modenblende (5.0) hinter dem Strahlteiler auf einen Fokus mit Durchmesser 0,18µm fokussiert. In einem Abstand von 80 mm unterhalb des Fokus war der Lasertransparenz(LT)-Messsensor positioniert. Die Testplatte wurde in einem Abstand von 2 mm oberhalb des LT-Messsensors positioniert. Die gesamte Messdauer betrug 30 s, wobei das Messergebnis in den letzten 5s ermittelt wurde. Die Signale von Referenz- und Messsensor wurden zeitgleich erfasst. Der Start der Messung erfolgte zeitgleich mit dem Einlegen der Probe.
Die Transmission und damit die Lasertransparenz ergab sich aus folgender Formel: LT = (Signal(Messsensor) / Signal(Referenzsensor)) x 100%. Durch diese Messweise wurden Schwankungen der Laseranlage und subjektive Ablesefehler ausgeschlossen.
Derartige lasertransparente Formkörper werden erfindungsgemäß zur Herstellung von Formkörpern mittels Laserdurchstrahlschweißverfahren verwendet.

Als laserabsorbierendes Formteil können generell Formkörper aus allen laserabsorbierenden Materialien eingesetzt werden. Dies können beispielsweise Verbundstoffe, Duroplaste oder bevorzugte Formkörper aus geeigneten thermoplastischen Formmassen sein. Geeignete thermoplastische Formmassen sind Formmassen, die eine ausreichende Laserabsorption im eingesetzten Wellenlängenbereich besitzen. Geeignete thermoplastische Formmassen können beispielweise bevorzugt Thermoplasten sein, die durch Zusatz von anorganischen Pigmenten wie beispielsweise Ruß und/oder durch Zusatz von organischen Pigmenten oder anderen Additiven laserabsorbierend sind. Geeignete organische Pigmente zur Erzielung von Laserabsorbtion sind beispielsweise bevorzugt IR-absorbierende organische Verbindungen, wie sie beispielsweise in DE 199 16 104 A1 beschrieben sind.

Gegenstand der Erfindung sind ferner Formkörper und/oder Formteilkombinationen, an die erfindungsgemäße Formteile mit dem Laserdurchstrahlschweißen verbunden wurden.

Erfindungsgemäße Formteile eignen sich hervorragend, um mit dem Laserdurchstrahlschweißverfahren an laserabsorbierende Formteile dauerhaft und stabil angebracht werden. Sie eignen sich daher insbesondere für Materialien für Deckel, Gehäuse, Anbauteile, Sensoren beispielsweise für Kfz-, Elektronik-, Telekommunikations-, Informationstechnologie-, Computer-, Haushalts-, Sport-, Medizin- oder Unterhaltungsanwendungen.

### Beispiele

Es wurden folgende Komponenten verwendet:
Komponente A/1
   Polyamid 6 mit einer Viskositätszahl VZ von 150 ml/g, gemessen als 0,5 gew.-%ige Lösung in 96 gew.-%iger Schwefelsäure bei 25 °C nach ISO 307 (Es wurde Ultramid® B27 der BASF SE verwendet.)
Komponente A/2
   PA 66 mit VZ von 150 ml/g (Ultramid® A27 der BASF SE)

### Materialien:

- B1: trans-1,4-Cyclohexyldiisocyanat (CAS 7517-76-2)
- B2: Hexamethylendiisocyanat (CAS 822-06-0)
- B3: Dicyclohexylmethan-4,4'-diisocyanat (CAS 5124-30-1)
- B4: 4,4'-Methylenbis(phenylisocyanat) (CAS 101-68-8)
- B5: Toluol 2,4-diisocyanat (CAS 584-84-9)
- B6: Cyclohexylisocyanat (CAS 3173-53-3)
- B7: 1,4-Phenylendiisocyanat (CAS 104-49-4)
- B8: Phenylisocyanat (CAS 103-71-9)
- B9: Isophorondiisocyanat (CAS 4098-71-9)

### Strukturformeln:

| | |
|---|---|
| B1 | 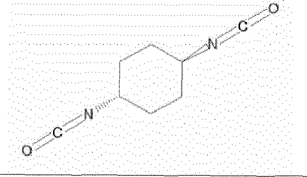 |
| B2 | 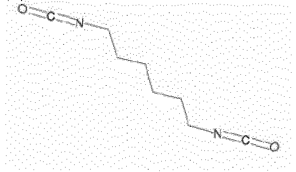 |
| B3 | 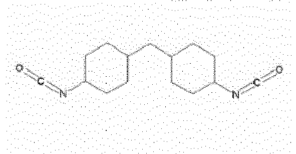 |
| B4 | 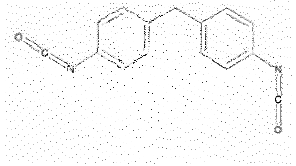 |
| B5 | 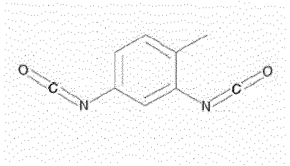 |
| B6 | 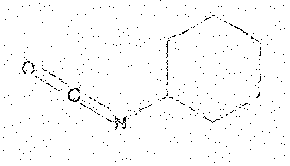 |
| B7 | 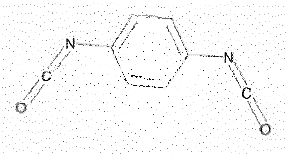 |
| B8 | 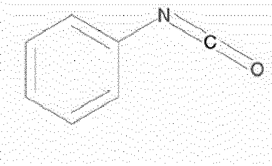 |
| B9 | 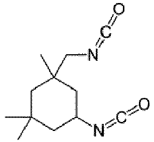 |

### Verarbeitung:

### Compounding - DSM:

Das Polyamidgranulat und die jeweiligen Isocyanate (1 Gew.%) wurden in eine Glasflasche eingewogen und anschließend in einem konischen Doppelschneckenextruder (DSM Xplore, 15cc) unter Stickstoff eincompoundiert. Das reine Polyamid wurde auf die gleiche Weise verarbeitet um die Referenzprobe zu erhalten. Es wurden folgende Parameter verwendet:
Verweilzeit: 3 min.
Gehäusetemperatur: 260°C
Schmelzetemperatur: 240°C -245°C
Drehzahl: 200 Upm

### Spritzguss - DSM:

Die Spritzgussverarbeitung der compoundierten Polymere wurde auf einer DSM-Micro-Injection Moulding Apparatur 10cc durchgeführt. Hierfür wurde der geschmolzene Compound unter Stickstoff direkt in den Zylinder der Spritzgussmaschine gefüllt. Die Schmelze wurde anschließend in eine polierte rechteckige Form mit den Maßen (30mm x 30mm x 1,27 mm) gespritzt. Es wurden folgende Parameter verwendet:
Mould: Plättchen poliert; 30mm x 30mm x 1,27 mm
Mouldtemperatur: 70°C
Zylindertemperatur: 260°C
Einspritzdruck: 10 - 12 bar
Messmethoden:

### Polymer Kristallisationstemperatur

Das Kristallisationsverhalten der Polymermischungen wird mittels dynamischer Differenzkaloriemetrie (DSC: Differential Scanning Calorimetrie) in an sich bekannter Weise bestimmt (ISO 11357-2:2013). Die Bestimmung erfolgt unter Stickstoff in offenen Aluminiumtiegeln bei einer Aufheizrate und Abkühlrate von 20 K/min. Nach dem ersten Aufheizen wird die Probe 5 min in der Schmelze gehalten um die thermische Vorgeschichte des Polymers zu löschen. Die DSC-Messung wird an ein- und derselben Probe zweckmäßigerweise ein- bis zweimal wiederholt, um eine definierte thermische Vorgeschichte des jeweiligen Polyamids sicherzustellen. Die Kristallisationstemperatur Tk wurden nach DIN EN ISO 11357-3 bestimmt. Die Kristallisationtemperatur Tk ist das exotherme Peakminimum der DSC Kurve beim ersten Abkühlen mit 20 K/min nach definierter thermischer Vorgeschichte.

### Optical characterization (Haze, Clarity):

Haze, Clarity und Transmission wurden mit einem haze-gard plus Messinstrument (BYK-G, Gardner®, illumination CIE-E) bei Raumtemperatur gemessen. Die Messung erfolgte nach ASTM D1003. Die Haze und Clarity Werte wurden 24 bis 48 h nach dem Spritzguss gemessen.

**Tabelle 1:**

| Zusammensetzung der Compounds | | | | | | | | | | | |
|---|---|---|---|---|---|---|---|---|---|---|---|
| Exp No | A/1 (Gew%) | A/2 (%) | B1 (%) | B2 (%) | B3 (%) | B4 (%) | B5 (%) | B6 (%) | B7 (%) | B8 (%) | B9 (%) |
| 1V | 100 | 0 | 0 | 0 | 0 | 0 | 0 | 0 | 0 | 0 | 0 |
| 2 | 99 | 0 | 1 | 0 | 0 | 0 | 0 | 0 | 0 | 0 | 0 |
| 3 | 99 | 0 | 0 | 1 | 0 | 0 | 0 | 0 | 0 | 0 | 0 |
| 4 | 99 | 0 | 0 | 0 | 0 | 0 | 0 | 0 | 0 | 0 | 0 |
| 5 | 99 | 0 | 0 | 0 | 1 | 0 | 0 | 0 | 0 | 0 | 0 |
| 6 | 99 | 0 | 0 | 0 | 0 | 1 | 0 | 0 | 0 | 0 | 0 |
| 7 | 99 | 0 | 0 | 0 | 0 | 0 | 1 | 0 | 0 | 0 | 0 |
| 8 | 99 | 0 | 0 | 0 | 0 | 0 | 0 | 1 | 0 | 0 | 0 |
| 9 | 99 | 0 | 0 | 0 | 0 | 0 | 0 | 0 | 1 | 0 | 0 |
| 10 | 99 | 0 | 0 | 0 | 0 | 0 | 0 | 0 | 0 | 1 | 0 |
| 11 | 99 | 0 | 0 | 0 | 0 | 0 | 0 | 0 | 0 | 0 | 1 |
| 12V | 0 | 100 | 0 | 0 | 0 | 0 | 0 | 0 | 0 | 0 | 0 |
| 13 | 0 | 99 | 1 | 0 | 0 | 0 | 0 | 0 | 0 | 0 | 0 |

**Tabelle 2:**

| Zusammensetzung | Haze [%] | Clarity [%] |
|---|---|---|
| 1V | 99.8 | 63.6 |
| 2 | 25.5 | 97.4 |
| 3 | 72.7 | 59.5 |
| 4 | 95.4 | 55.9 |
| 5 | 97.5 | 30.3 |
| 6 | 96.1 | 82.2 |
| 7 | 80.1 | 41.8 |
| 8 | 92.5 | 9.1 |
| 9 | 86.7 | 88.2 |
| 10 | 91.6 | 24.1 |
| 11 | 97.2 | 28.7 |
| 12V | 99.6 | 5.5 |
| 13 | 63.6 | 96.1 |

## Patentansprüche

1. Verwendung von thermoplastischen Formmassen, enthaltend
A) 30 bis 99 Gew.-% eines thermoplastischen Polyamids
B) 0,01 bis 10 Gew.-% eines organischen Isocyanates oder Diisocyanates oder deren Mischungen
C) 0 bis 60 Gew.-% weiterer Zusatzstoffe,
wobei die Summe der Gewichtsprozente A) bis C) 100 % ergibt,
zur Herstellung von Formkörpern jeglicher Art mit verbessertem Haze (gemessen nach ASTM D1003) und/oder verbesserter Clarity (gemessen nach ASTM D1003) und/oder erhöhter Lasertransparenz (gemessen bei einer Wellenlänge von 1064 nm mittels einer thermoelektrischen Leistungsmessung).

2. Verwendung nach Anspruch 1, wobei die Formmassen aus
A) 30 bis 99 Gew.-%
B) 0,01 bis 5 Gew.-%
C) 0 bis 50 Gew.-%
aufgebaut sind.

3. Verwendung nach den Ansprüchen 1 oder 2, enthaltend als organisches Isocyanat B) eine Verbindung der Formel R¹-N=C=O, wobei der Rest R¹ der Komponente B) für lineare C1-C14-Alkylreste, verzweigte C3 bis C12-Alkylreste, unsubstituierte oder substituierte C3 bis C14-Cycloalkylreste, unsubstituierte oder substituierte aromatische Reste mit 6 bis 20 C-Atomen steht.

4. Verwendung nach den Ansprüchen 1 bis 3, enthaltend als organisches Diisocyanat B) eine Verbindung der Formel O=C=N-R²-N=C=O,
wobei R² für lineare oder verzweigte C1 bis C14-Alkylenreste, unsubstituierte oder substituierte Cycloalkylenreste mit 3 bis 17 C-Atomen, substituierte oder unsubstituierte aromatische Reste mit 6 bis 20 C-Atomen steht.

5. Verwendung nach den Ansprüchen 1 bis 4, in denen die Komponente B) aus trans-1,4-Cyclohexyldiisocyanat (CAS 7517-76-2)
Hexamethylendiisocyanat (CAS 822-06-0)
Dicyclohexylmethan-4,4'-diisocyanat (CAS 5124-30-1)
4,4'-Methylenbis(phenylisocyanat) (CAS 101-68-8)
Toluol 2,4-diisocyanat (CAS 584-84-9)
Cyclohexylisocyanat (CAS 3173-53-3)
1,4-Phenylendiisocyanat (CAS 104-49-4)
Phenylisocyanat (CAS 103-71-9)
Isophorondiisocyanat (CAS 4098-71-9),
aufgebaut ist.

6. Verwendung nach den Ansprüchen 1 bis 5, wobei der Formkörper einen Haze-Wert aufweist, gemessen nach ASTM D1003, welcher mindestens 5% niedriger liegt verglichen mit einer Referenzpolymerzusammensetzung ohne die Komponente B), gemessen bei einer Probenkörperdicke (Platte) von 1.3 mm.

7. Verwendung nach den Ansprüchen 1 bis 6, wobei der Formkörper einen Clarity-Wert aufweist, gemessen nach ASTM D1003, welcher mindestens 5% höher liegt verglichen mit einer Referenzpolymerzusammensetzung ohne die Komponente B), gemessen bei einer Probenkörperdicke (Platte) von 1.3 mm.

8. Verwendung nach den Ansprüchen 1 bis 7, wobei der Formkörper eine Lasertransparenz aufweist, gemessen bei einer Wellenlänge von 1064 nm mittels einer thermoelektrischen Leistungsmessung, welche mindestens 1% höher liegt verglichen mit einer Referenzpolymerzusammensetzung ohne die Komponente B), gemessen bei einer Probenkörperdicke (Platte) von 1.3 mm.

9. Verwendung von transparenten Formkörpern gemäß den Ansprüchen 1 bis 8, zur Herstellung von Formkörpern mittels Laserdurchstrahlschweißen.

## Claims

1. The use of thermoplastic molding compositions comprising
A) from 30 to 99% by weight of a thermoplastic polyamide
B) from 0.01 to 10% by weight of an organic isocyanate or diisocyanate, or a mixture of these
C) from 0 to 60% by weight of other additional substances,
where the sum of the percentages by weight of A) to C) is 100%,
for the production of moldings of any type with improved haze (measured in accordance with ASTM D1003) and/or improved clarity (measured in accordance with ASTM D1003) and/or increased laser transparency (measured at a wavelength of 1064 nm by means of a thermoelectric power measurement).

2. The use according to claim 1, where the molding compositions are composed of
A) from 30 to 99% by weight
B) from 0.01 to 5% by weight
C) from 0 to 50% by weight.

3. The use according to claim 1 or 2, comprising a compound of the formula R¹-N=C=O) as organic isocyanate B), where the moiety R¹ of component B) represents linear C1-C14-alkyl moieties, branched C3 to C12-alkyl moieties, unsubstituted or substituted C3 to C14-cyclo-alkyl moieties, or unsubstituted or substituted aromatic moieties having from 6 to 20 carbon atoms.

4. The use according to claims 1 to 3, comprising a compound of the formula O=C=N-R²-N=C=O as organic diisocyanate B), where R² represents linear or branched C1 to C14- alkylene moieties, unsubstituted or substituted cycloalkylene moieties having from 3 to 17 carbon atoms, or substituted or unsubstituted aromatic moieties having from 6 to 20 carbon atoms.

5. The use according to claims 1 to 4 in which component B) is composed of
cyclohexyl trans-1,4-diisocyanate (CAS 7517-76-2)
hexamethylene diisocyanate (CAS 822-06-0)
dicyclohexylmethane 4,4'-diisocyanate (CAS 5124-30-methylenebis(phenyl 4,4'-diisocyanate) (CAS 101-68-toluene 2,4-diisocyanate (CAS 584-84-9)
cyclohexyl isocyanate (CAS 3173-53-3)
phenylene 1,4-diisocyanate (CAS 104-49-4)
phenyl isocyanate (CAS 103-71-9)
isophorone diisocyanate (CAS 4098-71-9).

6. The use according to claims 1 to 5, where the haze value of the molding, measured in accordance with ASTM D1003, is at least 5% lower than that of a reference polymer composition without component B), measured from a test sample (sheet) of thickness 1.3 mm.

7. The use according to claims 1 to 6, where the clarity value of the molding, measured in accordance with ASTM D1003, is at least 5% higher than that of a reference polymer composition without component B), measured from a test sample (sheet) of thickness 1.3 mm.

8. The use according to claims 1 to 7, where the laser transparency of the molding, measured at a wavelength of 1064 nm by means of a thermoelectric power measurement, is at least 1% higher than that of a reference polymer composition without component B), measured from a test sample (sheet) of thickness 1.3 mm.

9. The use of transparent moldings according to claims 1 to 8 for the production of moldings by means of laser transmission welding.

## Revendications

1. Utilisation de matériaux de moulage thermoplastiques, contenant :
A) 30 à 99 % en poids d'un polyamide thermoplastique,
B) 0,01 à 10 % en poids d'un isocyanate ou diisocyanate organique ou leurs mélanges,
C) 0 à 60 % en poids d'autres additifs,
la somme des pourcentages en poids de A) à C) étant de 100 %,
pour la fabrication de corps moulés de tout type présentant un trouble amélioré (mesuré selon ASTM D1003) et/ou une clarté améliorée (mesurée selon ASTM D1003) et/ou une transparence au laser augmentée (mesurée à une longueur d'onde de 1 064 nm au moyen d'une mesure de puissance thermoélectrique).

2. Utilisation selon la revendication 1, dans laquelle les matériaux de moulage sont formés par :
A) 30 à 99 % en poids,
B) 0,01 à 5 % en poids,
C) 0 à 50 % en poids.

3. Utilisation selon les revendications 1 ou 2, contenant en tant qu'isocyanate organique B) un composé de formule R¹-N=C=O, le radical R¹ du composant B) représentant des radicaux alkyle en C1-C14 linéaires, des radicaux alkyle en C3-C12 ramifiés, des radicaux cycloalkyle en C3-C14 non substitués ou substitués, des radicaux aromatiques non substitués ou substitués de 6 à 20 atomes C.

4. Utilisation selon les revendications 1 à 3, contenant en tant que diisocyanate organique B) un composé de formule O=C=N-R²-N=C=O,
dans laquelle R² représente des radicaux alkylène en C1 à C14 linéaires ou ramifiés, des radicaux cycloalkylène non substitués ou substitués de 3 à 17 atomes C, des radicaux aromatiques substitués ou non substitués de 6 à 20 atomes C.

5. Utilisation selon les revendications 1 à 4, dans laquelle le composant B) est formé par :
le diisocyanate de trans-1,4-cyclohexyle (CAS 7517-76-2),
le diisocyanate d'hexaméthylène (CAS 822-06-0),
le 4,4'-diisocyanate de dicyclohexylméthane (CAS 5124-30-1),
le bis(phénylisocyanate) de 4,4'-méthylène (CAS 101-68-8),
le 2,4-diisocyanate de toluène (CAS 584-84-9),
l'isocyanate de cyclohexyle (CAS 3173-53-3),
le diisocyanate de 1,4-phénylène (CAS 104-49-4),
l'isocyanate de phényle (CAS 103-71-9),
le diisocyanate d'isophorone (CAS 4098-71-9).

6. Utilisation selon les revendications 1 à 5, dans laquelle le corps moulé présente une valeur de trouble, mesurée selon ASTM D1003, qui est au moins 5 % inférieure en comparaison d'une composition polymère de référence sans le composant B), mesurée à une épaisseur d'éprouvette (plaque) de 1,3 mm.

7. Utilisation selon les revendications 1 à 6, dans laquelle le corps moulé présente une valeur de clarté, mesurée selon ASTM D1003, qui est au moins 5 % supérieure en comparaison d'une composition polymère de référence sans le composant B), mesurée à une épaisseur d'éprouvette (plaque) de 1,3 mm.

8. Utilisation selon les revendications 1 à 7, dans laquelle le corps moulé présente une transparence au laser, mesurée à une longueur d'onde de 1 064 nm au moyen d'une mesure de puissance thermoélectrique, qui est au moins 1 % supérieure en comparaison d'une composition polymère de référence sans le composant B), mesurée à une épaisseur d'éprouvette (plaque) de 1,3 mm.

9. Utilisation de corps moulés transparents selon les revendications 1 à 8 pour la fabrication de corps moulés par soudage laser.
